# NEW EUROPEAN PATENT SPECIFICATION

(11) **EP 1 208 864 B2**
(45) Date of publication and mention of the opposition decision: **06.06.2012**
(45) Mention of the grant of the patent: 28.03.2007
(21) Application number: 02001152.4
(22) Date of filing: 19.02.1999
(51) Int. Cl.: A61M 15/00, B05D 7/24, B65D 83/14

(54) **Improvements in drug delivery devices**
Vorrichtungen zur Medikamentverabreichung
Dispositifs pour administrer des médicaments

(30) Priority: 23.02.1998 GB 9803780; 24.04.1998 GB 9808804; 07.07.1998 GB 9814717
(43) Date of publication of application: 29.05.2002
(62) Divisional of application: 99905106.3
(73) Proprietor: GLAXO GROUP LIMITED, Greenford, Middlesex UB6 0NN (GB)
(72) Inventor: Warby, Richard John, Emneth, Norfolk PE14 8 AF (GB)
(74) Representative: Rice, Jason Neale

(56) References cited:
- WO-A-96/28367
- WO-A-96/32345
- WO-A-97/32672
- WO-A-97/47347
- WO-A1-96/32151
- US-A- 3 272 442
- US-A- 4 252 848
- US-A- 5 341 800

## Description

This invention relates to improvements in pressurised dispensing containers and particularly those for dispensing a metered dose of medicament.

In metered dose inhalers, an aerosol stream from a pressurised dispensing container is fired towards a patient or user of the inhaler into an air flow. The air flow is created by a user inhaling through a mouthpiece of the inhaler and the medicament is released into this air flow at a point between the air inlet holes and the mouthpiece.

Conventional metering valves as disclosed in WO-A-96/28367, for use with pressurised dispensing containers comprise a valve stem co-axially slidable within a valve member defining an annular metering chamber, and outer and inner annular seals operative between the respective outer and inner ends of the valve stem and the valve member to seal the metering chamber therebetween. The valve stem is hollow whereby in a non-dispensing position of the valve stem, the metering chamber is connected to the container and charged with product therefrom. The valve stem is movable against the action of a spring to a dispensing position wherein the metering chamber is isolated from the container and vented to atmosphere for the discharge of product.

Other drug delivery devices include apparatus in which capsules containing a powdered medicament are mechanically opened at a dispensing station where inhaled air subsequently entrains the powder, which is then dispensed through a mouthpiece.

A problem with all such drug delivery devices is that deposition of the medicament, or a solid component from a suspension of a particulate product in a liquid propellant, on the internal surfaces and other components of the devices occurs after a number of operation cycles and/or storage. This can lead to reduced efficiency of operation of the device and of the resulting treatment in that deposition of the product reduces the amount of active drug available to be dispensed.

Some prior art devices rely on the dispenser being shaken in an attempt to dislodge the deposited particles as a result of the movement of a liquid propellant and product mixture. However, whilst this remedy is effective within the body of the container itself, it is not effective for particles deposited on the inner surfaces of the metering chamber. As the size of the chamber is significantly smaller, the restricted flow of fluid in the metering chamber (caused by the tortuosity of the flow path through the chamber) means that the fluid in the metering chamber does not move with enough energy to adequately remove the deposited particles.

It is an object of the present invention to provide drug delivery devices in general in which the deposition of the product and active drug component is minimised.

According to the invention there is provided a method of producing a metering valve according to claim 1.

A particular embodiment of the present invention will now be described, by way of example only, with reference to the accompanying drawings, in which:
Figure 1 is a cross-sectional view through an inhaler; and
Figure 2 is a cross-sectional view of a metering valve produced with the method of the present invention.

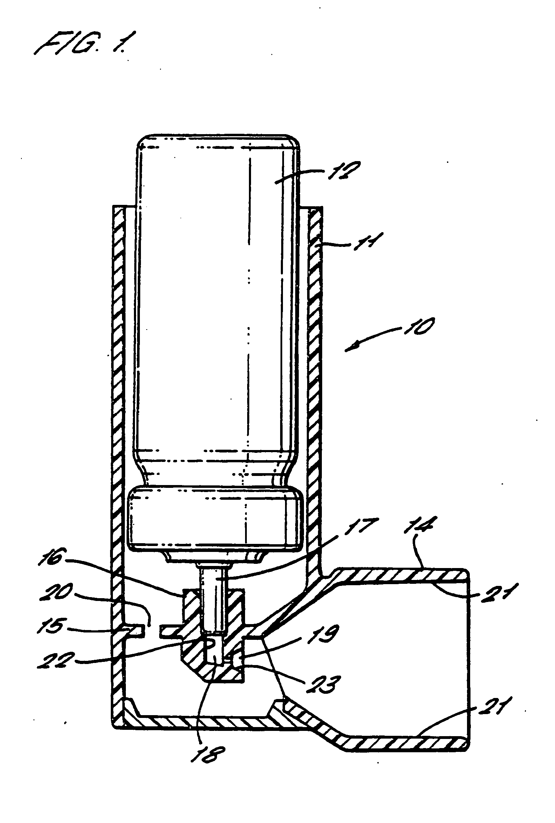

In Figure 1, an inhaler 10 for a product such as a medicament comprises a housing 11 for receiving a pressurised dispensing container 12 of a medicament and a mouthpiece 14 for insertion into the mouth of a user of the inhaler 10

The container 11 is generally cylindrical and open at its upper end. A lower wall 15 of the housing 11 includes an annular socket 16 for receiving the tubular valve stem 17 of the container 12. The socket 16 communicates via a duct 18 ending in an orifice 19 with the mouthpiece 14. The lower wall 15 also has holes 20 for allowing air to flow through the container housing 11 into the mouthpiece 14.

The mouthpiece 14 may be generally circular or shaped to fit the mouth and is connected to or forms a part of the housing 11.

In use, a patient or user holds the inhaler 10, usually in one hand, and applies his mouth to the mouthpiece 14. The user then inhales through the mouthpiece 14 and this creates an airflow through the cylindrical housing 11, from its open end around the dispensing container 12, through the holes 20 and into the mouthpiece 14. After the user has started inhaling through the mouthpiece 14, the container 12 is depressed downwardly onto its stem 17 to release a dose of medicament from the container 12. The dose of medicament is projected by the pressure in the container 12 via the duct 18 and through the orifice 19. It then mixes with the airflow through the mouthpiece 14 and is hence inhaled by the user.

In traditional inhalers, all of the components are plastic mouldings, which gives rise to the deposition problems described above. The particular problem areas in devices such as inhalers are the internal surfaces 21 of the mouthpiece 14, the internal surfaces 22 of the duct 18 and the walls 23 defining the orifice 19. In some inhalers 10, the diameter of at least a part of the duct 18 can be as little as 0.5mm and so any deposition on its internal surfaces 22 could lead to not only the problem of a reduction in active drug components being available, but also dispensing difficulties.

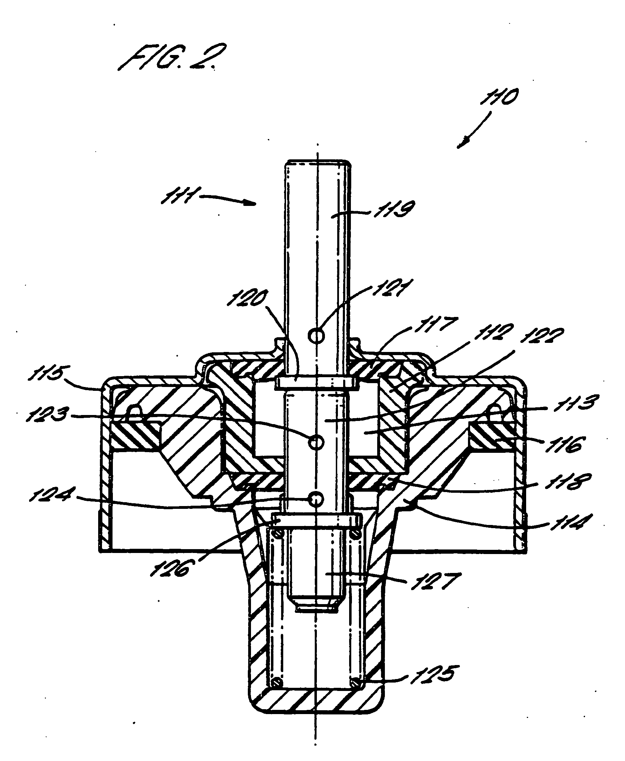

The metering valve 110 illustrated in Figure 2 includes a valve stem 111 which protrudes from and is axially slidable within a valve member 112, the valve member 112 and valve stem 111 defining therebetween an annular metering chamber 113. The valve member 112 is located within a valve body 114 which is positioned in a pressurised container (not shown) containing a product to be dispensed. The metering valve 110 is held in position with respect to the container by means of a ferrule 115 crimped to the top of the container and sealing being provided between the valve body 114 and container by an annular gasket 116.

An outer seal 117 and an inner seal 118 of an elastomeric material extend radially between the valve stem 111 and the valve member 112. The outer seal 117 is radially compressed between the valve member 112 and valve stem 111 so as to provide positive sealing contact, the compression being achieved by using a seal which provides an interference fit on the valve stem 111 and/or by the crimping of the ferrule 115 onto the pressurised container during assembly.

The valve stem 111 has an end 119 which protrudes from the valve member 112 and ferrule 115 which is a hollow tube and which is closed off by flange 120 which is located within the metering chamber 113. The hollow end 119 of valve stem 111 includes a discharge port 121 extending radially through the side wall of the valve stem 111. The valve stem 111 further has an intermediate section 122, which is also hollow and defining a central passage and which has a pair of spaced radial ports 123, 124 which are interconnected through a central cavity.

A spring 125 extends between a second flange 126, separating the intermediate section 122 of the valve stem 111 and an inner end 127 of the valve stem 111, and an end of the valve body 114 to bias the valve stem 111 in a non-dispensing position in which the first flange 120 is held in sealing contact with the outer seal 117. The second flange 126 is located outside the valve member 112, but within the valve body 114.

The metering chamber 113 is sealed from the atmosphere by the outer seal 117, and from the pressurised container to which the valve 110 is attached by the inner seal 118. In the illustration of the valve 110 shown in Figure 1 radial ports 123, 124, together with the central cavity in the intermediate section 122 of the valve member 111 connect the metering chamber 113 with the container so that in this non-dispensing condition the metering member 113 will be charged with product to be dispensed.

Upon depression of the valve stem 111 relative to the valve member 112 so that it moves inwardly into the container, the radial port 124 is closed off as it passes through the inner seal 118, thereby isolating the metering chamber 113 from the contents of the pressurised container. Upon further movement of the valve stem 111 in the same direction to a dispensing position the discharge port 121 passes through the outer seal 117 into communication with the metering chamber 113. In this dispensing position the product in the metering chamber 113 is free to be discharge to the atmosphere via the discharge port 121 and the cavity in the hollow end 119 of the valve stem 111.

When the valve stem 111 is released, the biasing of the return spring 125 causes the valve stem 111 to return to its original position. As a result the metering chamber 113 becomes recharged in readiness for further dispensing operations.

The component parts of conventional drug dispensing devices, such as valve members, valve stems, inhaler housings and so on, are generally formed as single mouldings from material such as acetal, polyester or nylon which are prone to the deposition problems described above. Although in some cases it might be possible to include a separate liner of a material such as a fluoropolymer, ceramic or glass to line a portion of the area in which deposition problems occurs, this requires the redesign or modification of mouldings and mould tools so that the components can accommodate such liners.

In the present invention we propose a solution in which the component parts of the pressurised dispensing container are made by conventional tooling and moulds from the traditional materials listed above. In the metering valve according to the present invention, the valve member is made of a plastic and is alone then subjected to a cold plasma polymerisation treatment of one or more monomers which is a "hydrophobic" treatment which creates a very thin layer of thickness in the range of 0.005 to 0.5 microns of a cold plasma polymerised fluorinated hydrocarbon bonded to the surface of the valve member which significantly reduces the deposition of active drugs on the relevant surfaces due to factors such as anti-frictional and waterproof characteristics and low surface energy.

The preferred monomers to use in this process are perfluoro-cyclohexane or perfluoro-hexane which would create a thin layer of plasma polymerised fluoro-cyclohexane or fluoro-hexane on the appropriate surface. Other fluorinated hydrocarbons may also be used, such as tetrafluoroethylene (TFE), trifluoroethylene, vinylidene fluoride and vinyl fluoride. The two monomers fluoroethylene and fluoropropylene may also be used to form the copolymer fluorinated ethylenepropylene (FEP).

The process is known as "cold plasma" treatment as the temperature within the body of the plasma is ambient. Thus thermoplastic materials such as polybutylene terephthalate (PBT), nylon, acetal and tetrabutyrene terephthalate (TBT) can be treated without fear of thermal damage. The treatment is a vacuum procedure in which the valve members are placed inside a chamber which is evacuated to less than 0.005 Torr. One or more monomers are introduced to the chamber at a controlled rate and a 13.56 MHZ r.f. signal is applied to an external antenna. The plasma is ignited within the chamber and maintained for a given time at the preselected power setting. At the end of the treatment the plasma is extinguished, the chamber flushed and the valve members retrieved. As a result a thin layer having a thickness in the range of 0.005 to 0.5 microns of the plasma polymerised material is intimately bonded to the surface of the valve member.

Either an entire valve member or just the surface of a valve member which would come into contact with the medicament during actuation could be treated according to the present invention. In the metering valve of Figure 2, the valve member 112 alone is treated.

## Claims

1. A method of producing a metering valve (110) for use with a pressurised dispensing container (12) for dispensing medicament from said container, the method comprising the steps of providing a valve stem (111), a valve member (112) and outer and inner annular seals (117, 118), and assembling the valve so that:-
- the valve stem is co-axially slidable within the valve member,
- said valve member (112) and valve stem (111) define an annular metering chamber (113) with a surface of the valve member (112) forming an internal surface of the metering chamber (113) which, in use, comes into contact with medicament; and
- the outer and inner annular seals (117, 118) are operative between the respective outer and inner ends of the valve member (112) and the valve stem (111) to seal the annular metering chamber (113) therebetween,
**characterised by** said valve member (112) being made of a plastic, by subjecting the surface of the valve member (112) to a cold plasma polymerisation treatment of one or more monomers to bond to the surface a layer of a cold plasma polymerised fluorinated hydrocarbon, by the layer having a thickness in the range of 0.005-0.5 microns, and by the valve member (112) alone being treated.

2. Method as claimed in claim 1, wherein the valve member is made from a material selected from the group consisting of acetal, polyester and nylon.

3. Method as claimed in claim 1 or 2, in which the one or more monomers for cold plasma polymerisation are selected from the group of materials comprising perfluoro-cyclohexane, perfluoro-hexane, tetrafluoroethylene, trifluoroethylene, vinylidene fluoride, vinylfluoride, fluoroethylene and fluoropropylene.

## Patentansprüche

1. Verfahren zur Herstellung eines Dosierventils (110) zur Verwendung mit einem unter Druck gesetzten Abgabebehälter (12) zur Abgabe eines Medikaments aus dem Behälter, wobei das Verfahren die Schritte des Vorsehens eines Ventilschafts (111), eines Ventilelements (112) und äußerer und innerer ringförmiger Dichtungen (117, 118), und des Zusammenbauens des Ventils umfasst, so dass:
- der Ventilschaft in dem Ventilelement koaxial gleitbar ist,
- das Ventilelement (112) und der Ventilschaft (111) eine ringförmige Messkammer (113) definieren, wobei eine Oberfläche des Ventilelements (112) eine innere Oberfläche der Messkammer (113) ausbildet, die, bei der Verwendung, mit einem Medikament in Kontakt gelangt, und
- wobei die äußeren und inneren ringförmigen Dichtungen (117, 118) zwischen den jeweiligen äußeren und inneren Enden des Ventilelements (112) und des Ventilschafts (111) funktionsfähig sind, um die ringförmige Messkammer (113) dazwischen abzudichten,
**dadurch gekennzeichnet, dass** das Ventilelement (112) aus einem Kunststoff hergestellt ist, dass die Oberfläche des Ventilelements (112) einer Kaltplasma-Polymerisationsbehandlung mit einem oder mehreren Monomeren ausgesetzt wird, um eine Schicht aus einem kaltplasmapolymerisierten fluorierten Kohlenwasserstoff an die Oberfläche zu binden, dass die Schicht eine Dicke in dem Bereich von 0,005 bis 0,5 Mikrometer aufweist, und dass allein das Ventilelement (112) behandelt wird.

2. Verfahren nach Anspruch 1, bei dem das Ventilelement aus einem Material hergestellt ist, das aus der Gruppe ausgewählt wird, die aus Acetal, Polyester und Nylon besteht.

3. Verfahren nach Anspruch 1 oder 2, bei dem die einen oder mehreren Monomere für die Kaltplasma-Polymerisation aus der Gruppe von Materialien ausgewählt werden, die Perfluorcyclohexan, Perfluorhexan, Tetrafluorethylen, Trifluorethylen, Vinylidenfluorid, Vinylfluorid, Fluorethylen und Fluorpropylen umfassen.

## Revendications

1. Procédé pour produire une soupape de dosage (110) prévue pour une utilisation avec un récipient de distribution sous pression (12) afin de distribuer un médicament à partir dudit récipient, le procédé comprenant les étapes suivantes :
préparer une tige de soupape (111), un élément de soupape (112) et des joints annulaires externe et interne (117, 118), et assembler la soupape de telle manière que :
- la tige de soupape puisse coulisser coaxialement à l'intérieur de l'élément de soupape ;
- lesdits élément de soupape (112) et tige de soupape (111) définissent une chambre de dosage annulaire (113), avec une surface de l'élément de soupape (112) formant une surface intérieure de la chambre de dosage (113), et ladite surface, en utilisation, vienne en contact avec le médicament ; et
- les joints annulaires externe et interne (117, 118) fonctionnent entre les extrémités extérieure et intérieure respectives de l'élément de soupape (112) et la tige de soupape (111) afin de créer une étanchéité entre eux dans la chambre de dosage annulaire (113) ;
**caractérisé en ce que** ledit élément de soupape (112) est en matière plastique et **en ce que** la surface de l'élément de soupape (112) est soumise à un traitement de polymérisation sous plasma froid d'un ou de plusieurs monomères afin de lier sur la surface une couche d'un hydrocarbure fluoré polymérisé sous plasma froid, **en ce que** la couche présente une épaisseur comprise dans une plage égale à 0,005 - 0,5 micromètres, et **en ce que** l'élément de soupape (112) seul est traité.

2. Procédé selon la revendication 1, dans lequel l'élément de soupape est en un matériau choisi parmi le groupe comprenant l'acétal, le polyester et le nylon.

3. Procédé selon la revendication 1 ou 2, dans lequel ledit ou lesdits monomères destinés à la polymérisation sous plasma froid sont choisis parmi le groupe de matériaux comprenant : perfluorocyclohexane, perfluorohexane, tétrafluoroéthylène, trifluoroéthylène, fluorure de vinylidène, fluorure de vinyle, fluoroéthylène et fluoropropylène.
